Europäisches Patentamt

European Patent Office

Office européen des brevets

(11) Veröffentlichungsnummer: **0 126 282**
A2

## (12) EUROPÄISCHE PATENTANMELDUNG

(21) Anmeldenummer: **84104173.4**

(22) Anmeldetag: **13.04.84**

(51) Int. Cl.³: **F 24 F 3/14**

(30) Priorität: **27.04.83 DE 3315231**

(43) Veröffentlichungstag der Anmeldung: **28.11.84**
**Patentblatt 84/48**

(84) Benannte Vertragsstaaten: **AT BE CH DE FR GB IT LI LU NL SE**

(71) Anmelder: **INTERATOM Internationale Atomreaktorbau GmbH, Friedrich-Ebert-Strasse, D-5060 Bergisch Gladbach 1 (DE)**

(72) Erfinder: **Tittizer, Gabriel, Dipl.-Ing., In den Erlen 22, D-5068 Odenthal-Eikamp (DE)**
Erfinder: **Hahn, Franz-Josef, Dipl.-Ing., Viehtrifft 21, D-5000 Köln 91 (DE)**

(74) Vertreter: **Mehl, Ernst, Dipl.-Ing. et al, Postfach 22 01 76, D-8000 München 22 (DE)**

(54) **Verfahren und Vorrichtung zur kontinuierlichen Herstellung definierter, sehr geringer Feuchten in einem Trägergas.**

(57) Gemäß der vorliegenden Erfindung wird eine Vorrichtung zur Herstellung definierter Feuchten in einem Trägergas vorgeschlagen, welche einen kontinuierlichen Betrieb mit hoher Genauigkeit ermöglicht und schnell auf verschiedene Konzentrationen einstellbar ist. Dazu wird das Trägergas in einem geschlossenen Kreislauf, insbesondere bei geringem Überdruck geführt und in einem Kühlbad auf der gewünschten Temperatur bis zu dem entsprechenden Wasserdampf-Partialdruck angefeuchtet bzw. entfeuchtet. Das Trägergas wird mit definiertem Wasserdampf-Partialdruck im weiteren Verlauf des Kreislaufes in einem Aufwärmabschnitt (12) aufgewärmt, wobei zwischen Kühlbereich (4) und Aufwärmabschnitt (12) mit Hilfe eines Heizdrahtes (15) ein Temperatursprung aufrecht erhalten wird. Durch diese Anordnung wird erreicht, daß es zwischen kaltem Bereich (4) und warmem Bereich (12) keine Eisgrenze mehr gibt, die durch ihre Verschiebung Ungenauigkeiten in der Messung hervorrufen könnte. Entsprechend geringer ist die Einstellzeit für verschiedene Konzentrationen.

83 P 6710 E  0126282

**INTERATOM**    24.673.0

Internationale Atomreaktorbau GmbH

D-5060 Bergisch Gladbach 1

Verfahren und Vorrichtung zur kontinuierlichen Herstellung definierter, sehr geringer Feuchten in einem
Trägergas

Die vorliegende Erfindung betrifft ein Verfahren nach
dem Oberbegriff des Hauptanspruchs und eine Vorrichtung
zur Durchführung dieses Verfahrens. Für die Einstellung
von definierten Feuchten ist es bekannt, daß über Eis
bei jeder Temperatur ein genau definierter Wasserdampf-
Partialdruck herrscht. Kühlt man ein Gas mit hohem
Feuchtigkeitsgehalt unter $0^o$ C ab, so friert die überschüssige Wassermenge aus, und es stellt sich für jede
Temperatur ein bestimmter Sättigungs-Partialdruck über
dem Eis ein. Der Partialdruck kann daher über eine genaue Temperaturmessung präzise bestimmt werden.

Es sind verschiedene Geräte, die diesen Effekt ausnutzen
bekannt, wobei sich jedoch Schwierigkeiten bei der kontinuierlichen Herstellung eines Trägergases mit definiertem Feuchtegehalt ergeben haben. Insbesondere waren bei
bisherigen Anordnungen lange Einstellzeiten nötig und der
Feuchtegehalt ließ sich bei Durchsatzschwankungen oder
Änderung der Umgebungstemperatur nicht konstant halten.

Aufgabe der vorliegenden Erfindung ist ein Verfahren zur
kontinuierlichen Herstellung eines Trägergases mit definierter Feuchte sowie eine Vorrichtung zur Durchführung
des Verfahrens, bei denen die beschriebenen Nachteile
vermieden werden. Insbesondere soll das Gerät im Betrieb
robust sein und schnell auf verschiedene Konzentrationen

31.03.83 Nw/Pa

83 P 6710 E    0126282

-2-    24.673.0

eingestellt werden können. Darüberhinaus soll der Feuchtegehalt auch bei leichten Durchsatzschwankungen des Trägergases nicht    verändert werden.

Zur Lösung dieser Aufgabe wird ein Verfahren nach dem
Hauptanspruch vorgeschlagen. Dazu wird das in einem geschlossenen Kreislauf, insbesondere bei geringem Überdruck geführte Trägergas in einem Kühlbad auf die dem
gewünschten Wasserdampf-Partialdruck entsprechende Temperatur abgekühlt, wodurch sich die entsprechende Feuchtekonzentration einstellt. Das Trägergas wird mit diesem
definierten Wasserdampf-Partialdruck im weiteren Verlauf
des Kreislaufes in einem Aufwärmabschnitt wieder aufgewärmt, wobei zwischen Kühlbereich und Aufwärmabschnitt
ein Temperatursprung aufrecht erhalten wird. Dabei liegt
der erfindungsgemäßen Lösung die Erkenntnis zugrunde, daß
die bisherigen Ungenauigkeiten bei der Einstellung von
geringen Feuchten gerade durch den Übergangsbereich zwischen dem Kühlbad und dem normalerweise auf Raumtemperatur befindlichen Meßpunkt verursacht wurden. An irgendeiner Stelle dieses früher notwendigerweise immer vorhandenen Übergangsbereiches befindet sich nämlich die Eisgrenze, welche sich bei kleinen Temperaturänderungen oder
Durchsatzschwankungen verschiebt und dadurch Feuchtigkeit
freisetzt bzw. bindet. Der wesentliche Schritt des erfindungsgemäßen Verfahrens liegt demgemäß in dem Temperatursprung zwischen Kühlbereich und Aufwärmabschnitt, welcher
dazu führt, daß überhaupt keine Eisgrenze vorhanden ist,
welche sich verschieben könnte.

Eine entsprechende Vorrichtung zur Durchführung des Verfahrens wird im Anspruch 2 vorgeschlagen. Danach ist ein
geschlossener Gaskreislauf, insbesondere unter leichtem
Überdruck vorhanden, der teilweise in einem thermostatisierten Kühlbad verläuft, wobei insbesondere eine Wasser-
dampfdruck-Ausgleichsvorrichtung und ein Beruhigungsgefäß

-3-                    24.673.0

im Kühlbad angeordnet sind. In der Ausgleichsvorrichtung wird entsprechend der vorherrschenden Temperatur das durchgeleitete Trägergas abgesättigt; dies geschieht entweder durch Anfeuchten oder Ausfrieren in Abhängigkeit vom Zustand des eingeleiteten Gases. Das nachgeschaltete Beruhigungsgefäß soll verhindern, daß Wasserpartikel (z.B. in Form von Nebel) in den Auslaß der Vorrichtung gelangen. In das Beruhigungsgefäß ragt von außerhalb des Kühlbades ein beheizbares Aufwärmrohr hinein, welches im Inneren des Beruhigungsgefäßes endet und als Auslaß für das Trägergas dient. Bei dieser Anordnung befinden sich die Ausgleichsvorrichtung und das Beruhigungsgefäß mit seinen Wänden auf der Temperatur des Kühlbades. Dadurch bildet sich über dem dort vorhandenen Eis exakt der der Temperatur entsprechende Wasserdampf-Partialdruck heraus. Das beheizbare Aufwärmrohr hingegen wird auf seiner vollen Länge durch die Heizung auf deutlich höherer Temperatur gehalten, so daß in seinem Inneren an keiner Stelle eine Eisbildung zu befürchten ist. Der deutliche Temperatursprung von den Wänden des Beruhigungsgefäßes zum beheizten Aufwärmrohr bewirkt, daß keine Eisgrenze vorhanden ist, welche sich bei leichten Betriebsänderungen verschieben könnte. Im gesamten Aufwärmrohr ist das Gasgemisch weit vom Taupunkt entfernt, so daß kleinere Störungen sich nicht durch Ausfrieren des Wassers bemerkbar machen können.

Im weiterer Ausgestaltung der Erfindung wird im Anspruch 3 vorgeschlagen, daß das Aufwärmrohr durch einen in seinem Inneren verlaufenden Heizdraht beheizt werden soll, wobei der Heizdraht im wesentlichen ohne Wandberührung in der Mitte des Aufwärmrohres verlaufen soll. Diese Ausgestaltung ist deshalb besonders günstig, weil die Wände des Aufwärmrohres bei dieser Anordnung im wesentlichen durch Wärmestrahlung des Heizdrahtes eisfrei gehalten werden und nicht wie bei anderen denkbaren Heizsystemen durch Wärmeleitung. Diese Art der Beheizung des Aufwärmrohres bewirkt auch daß der Bereich des Aufwärmrohres, welcher

83 P G 7 P 0 1 2 6 2 8 2

durch das Kühlmittel hindurchführt, trotzdem innen eisfrei gehalten werden kann, was entsprechend den obigen Ausführungen notwendig ist.

In spezieller Ausgestaltung der Erfindung wird im Anspruch 4 vorgeschlagen, daß der Heizdraht durch kleine Distanzstücke zumindest im Bereich unterhalb des Kühlmittelspiegels auf Abstand von der Wand des Aufwärmrohres gehalten wird. Dies führt zu einer gleichmäßigen Aufwärmung aller Wände des Aufwärmrohres und verhindert eine ungleichmäßige Aufwärmung durch Wärmeleitung.

In zusätzlicher Ausgestaltung der Erfindung wird im Anspruch 5 vorgeschlagen, daß der Heizdraht, bzw. seine Zuleitung, an einer Abzweigung oberhalb des Kühlmittelspiegels dicht durch die Wand des Aufwärmrohres nach außen geführt wird. Da der Heizdraht mit einer Stromversorgung verbunden werden muß, andererseits das geschlossene System aber dicht abgeschlossen bleiben soll, ist diese Art der Zuführung besonders geeignet. Die Strömung des Trägergases an dem Heizdraht vorbei wird dadurch nicht beeinträchtigt.

Im Anspruch 6 wird weiterhin vorgeschlagen, daß die Durchführung des Aufwärmrohres in das Beruhigungsgefäß möglichst kurz und/oder schlecht wärmeleitend ausgeführt ist. Da diese Durchführung sich unterhalb des Kühlmittelspiegels befindet, liegt dort die kritischste Stelle für eine Eisbildung im Inneren des Aufwärmrohres, da dort durch Wärmeleitung die niedrigsten Temperaturen herrschen. Die vorgeschlagenen Maßnahmen in Verbindung mit einer entsprechenden Heizleistung des Heizdrahtes gewährleisten aber, daß auch dort im Inneren des Aufwärmrohres eine Eisbildung nicht zu befürchten ist.

Zusätzlich wird im Anspruch 7 vorgeschlagen, daß das Aufwärmrohr aus einem schlecht wärmeleitenden Material bestehen soll. Bei dieser Ausführung sind die geringsten Wärmeverluste an das Kühlmittel zu befürchten und die in das Kühlmittel abgeführte Heizleistung ist entsprechend gering, wodurch der Kühlkreislauf nicht übermäßig belastet wird.

In besonderer Ausgestaltung der Erfindung wird im Anspruch 8 vorgeschlagen, daß der Heizdraht etwas über das Ende des Aufwärmrohres hinaus in das Innere des Ausfriergefäßes ragen soll. Diese Maßnahme stellt sicher, daß auch am untersten Ende des Aufwärmrohres keine Eisbildung mit einer möglichen Verschiebung der Eisgrenze auftreten kann.

Im Anspruch 9 werden weitere Ausgestaltungen der Erfindung beschrieben, welche bei einem erfindungsgemäßen geschlossenen Kreislauf besonders günstig sind. Dazu werden in dem Kühlbad nebeneinander eine Kühlschlange, eine Wasserdampfdruck-Ausgleichsvorrichtung und ein Beruhigungsgefäß mit einem als Auslaß dienenden Aufwärmrohr angeordnet, welche nacheinander vom Trägergas durchströmbar sind. Der geschlossene Kreislauf hat den Vorteil, daß das Gas nach einigen Umläufen schon beim Eintritt in das Kühlmittel praktisch schon die gewünschte Feuchte aufweist und nur noch kleine Korrekturen beim nächsten Durchlauf erfolgen müssen. Dazu wird das Trägergas in einer Kühlschlange vorgekühlt und anschließend in einen ebenfalls auf der Kühlbad-Temperatur befindlichen Behälter mit einem feuchten Pulver geleitet. Dieses angefeuchtete Pulver, vorzugsweise z.B. Aluminiumoxid, befeuchtet das Trägergas, falls dessen Feuchte zu niedrig ist und dient als Wasservorrat für gegebenenfalls einzustellende höhere Feuchtigkeitskonzentrationen. Im gleichen Behälter wird jedoch auch, sollte das eingeleitete Trägergas der Kühlbad-Temperatur entsprechend noch zu feucht sein, überschüssige Feuchte ausgeforen, soweit dies noch nicht in der vorgeschalteten Kühlschlange geschehen ist. Dieses Gefäß erfüllt also eine Ausgleichsfunktion derart, daß am Ausgang
des Gefäßes der Wasserdampf-Partialdruck des Trägergases
exakt dem Sättigungsdruck bei Kühlbad-Temperatur entspricht.
Das Trägergas wird entweder angefeuchtet oder überschüssige
Feuchte ausgefroren, je nach Zustand des ankommenden Gases.
Die Wände des Beruhigungsgefäßes und insbesondere der Boden
können dabei mit einer dünnen Eisschicht bedeckt sein.
Durch das oben schon beschriebene Aufwärmrohr gelangt das
Trägergas dann wieder aus dem Kühlbadbereich in den Normaltemperaturbereich, wo es beispielsweise zur Kalibrierung
von Feuchtefühlern verwendet werden kann.

Im Anspruch 10 werden weitere besonders günstige Merkmale
der Ausgleichsvorrichtung beschrieben. Danach mündet ein
Gaseinlaßrohr oberhalb der Füllung in dem mit feuchtem Pulver gefüllten Behälter der Ausgleichsvorrichtung. Der Gasauslaß besteht aus einem in der Füllung endenden Rohr,
welches perforiert ist. Das Trägergas muß also durch das
feuchte Pulver hindurchströmen, wodurch wegen der großen
Oberflächen eine schnelle und genaue Einstellung des
Feuchtegehaltes bewirkt wird.

In weiterer Ausgestaltung der Erfindung wird im Anspruch 11
eine günstige Ausgestaltung des Gaseinlasses in das Beruhigungsgefäß beschrieben. Dieser Gaseinlaß besteht aus einem
perforierten Rohr, welches etwas oberhalb des Bodens in das
Beruhigungsgefäß hineinragt. Bei dieser Ausführung ist es
möglich, daß sich unterhalb des Rohres eine Wasserschicht
bilden kann, ohne daß der Weg des Trägergases dadurch behindert wird.

Außerhalb des Kühlbades befinden sich gemäß dem Anspruch
12 Hilfsgeräte zum Betrieb des Kreislaufes, sowie eine oder mehrere Anschlußstellen für Feuchtemeßgeräte. Beim Betrieb eines geschlossenen Kreislaufes ist es nötig, Hilfsgeräte wie Pumpen und Meßgeräte zu betreiben, welche vernünftigerweise zugänglich außerhalb des Kühlbades angeordnet werden.

Im Anspruch 13 werden dementsprechend einige der notwendigen Hilfsgeräte zum Betrieb des Kreislaufes beschrieben. Es sind dies eine Pumpe mit nachgeordnetem Dämpfungsgefäß zur Pulsationsdämpfung, ein Ausgleichsgefäß zur Aufrechterhaltung eines leichten Überdruckes und Meßgeräte für Durchfluß und Druck.

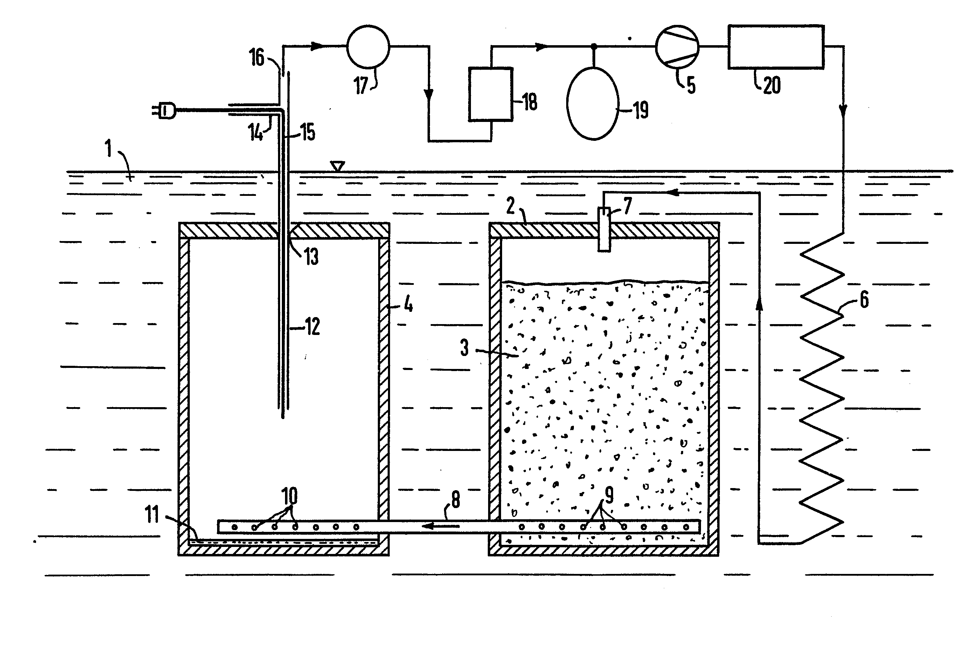

Die erfindungswesentlichen Merkmale sind anhand eines Ausführungsbeispieles halbschematisch in der Zeichnung dargestellt.

In einem thermostatisierten Kühlbad, dessen präzise Meß- und Regeleinrichtung nicht dargestellt ist, befindet sich eine Ausgleichsvorrichtung 2 mit einer feuchten Pulverfüllung 3, sowie ein Beruhigungsgefäß 4. Eine Membranpumpe 5 fördert Trägergas durch eine ebenfalls in dem Kühlbad angeordnete Kühlschlange 6 zum Einlaß 7 des pulvergefüllten Behälters, welcher als Wasserdampfdruck-Ausgleichsvorrichtung dient. Durch eine Rohrleitung 8, welche in die Pulverfüllung des Behälters 2 hineinragt und dort perforiert 9 ist, wird das Trägergas weitergeleitet. Die Leitung 8 führt in das Beruhigungsgefäß 4 hinein und endet in diesem etwas oberhalb des Gefäßbodens, wobei das Ende der Rohrleitung 8 in dem Beruhigungsgefäß 4 ebenfalls perforiert 10 ist. Bei dieser Anordnung ist es möglich, daß sich unterhalb der Einmündung dieser Leitung eine Eisschicht 11 bildet, ohne daß der Weg des Trägergases behindert wird. In dem Beruhigungsgefäß 4 stellt sich der der Temperatur des Kühlbades entsprechende Wasserdampf-Partialdruck ein und das definierte Gasgemisch wird durch das Aufwärmrohr 12 nach außen geleitet. Dieses Aufwärmrohr 12 ist an einer Durchführung 13 durch die Wand des Beruhigungsgefäßes 4 hindurchgeführt, wobei diese Durchführung eine möglichst geringe Wärmeleitung haben soll. An einer Abzweigung 14 ist ein Heizdraht 15 dicht in das Aufwärmrohr hineingeführt, in welchem er bis zum Ende des Aufwärmrohres verläuft. Die Heizleitung des Heizdrahtes ist so bemessen, daß das Innere des Aufwärmrohres bis zu seinem Ende überall eisfrei gehalten werden kann. Auf diese Weise gibt es keine Eisgrenze zwischen kaltem und warmem Bereich, welche sich bei Betriebsänderungen verschieben könnte.

Das Trägergas mit der nun exakt bekannten Feuchte kann nun, je nach Wunsch verwendet werden. Im allgemeinen wird im außerhalb des Kühlbades liegenden Kreislauf ein Feuchtemeßgerät installiert sein, welches mit Hilfe der beschriebenen Vorrichtung geeicht werden soll. Es können auch mehrere Meßgeräte im Vergleich betrieben werden. Das kalibrierte Gas wird dementsprechend durch ein Leitungssystem mit einer oder mehreren Anschlußstellen 17 für Feuchtefühler geleitet. Im weiteren Verlauf des Kreislaufes ist ein Strömungsmesser 18 angeordnet, welcher die Überwachung der Betriebsbedingungen ermöglicht. Da auch schon winzige Lecks Feuchtigkeit aus der Umgebungsluft in das System eindringen lassen würden, wird angestrebt, das System mit einem ganz geringen Überdruck zu betreiben, wodurch geringfügige Lecks keine Störeinflüsse ausüben. Im einfachsten Fall kann dieser Überdruck durch einen als Ausgleichsgefäß dienenden Gummiballon 19 aufrecht erhalten werden. Ein solcher Gummiballon kann im geschlossenen System auch die bei unterschiedlichen Temperaturen auftretenden Volumenänderungen auffangen. Darüberhinaus gibt sein Schrumpfen Hinweise auf eventuell vorhandene Lecks im System. Bei Verwendung einer Membranpumpe 5 zur Förderung des Trägergases im Kreislauf empfiehlt es sich, zur Dämpfung von Pulsationen ein Ausgleichsgefäß 20 in den Kreislauf einzubauen. Selbstverständlich können weitere Meßgeräte oder spezielle Entnahmestellen für das angefeuchtete Trägergas vorgesehen werden. Mit der beschriebenen Anordnung lassen sich bei entsprechend präzisier Thermostatisierung und Temperaturmessung des Kühlbades, welches beispielsweise mit Alkohol gefüllt sein könnte, genau definierte Feuchten in der Größenordnung von 1 ppm einstellen.

INTERATOM          −10−          24.673.0
Internationale Atomreaktorbau GmbH
D-5060 Bergisch Gladbach 1

Verfahren und Vorrichtung zur kontinuierlichern Herstellung definierter, sehr geringer Feuchten in einem Trägergas

Patentansprüche

1. Verfahren zur kontinuierlichen Herstellung definierter, sehr geringer Feuchten in einem Trägergas, wobei in einem Kühlbad (1) das angefeuchtete Trägergas auf die bestimmte Temperatur abgekühlt wird, bei der der gewünschte Wasserdampf-Partialdruck herrscht, g e k e n n z e i c h n e t   d u r c h   folgende Merkmale:

   a) Das Trägergas wird in einem geschlossenen Kreislauf, insbesondere bei geringem Überdruck geführt.

   b) Das Trägergas wird in einem Kühlbad (1) auf der gewünschten Temperatur bis zu dem entsprechenden Wasserdampf-Partialdruck angefeuchtet bzw. entfeuchtet.

   c) Das Trägergas wird mit definiertem Wasserdampf-Partialdruck im weiteren Verlauf des Kreislaufes in einem Aufwärmabschnitt (12) aufgewärmt, wobei zwischen Kühlbereich (4) und Aufwärmabschnitt (12) ein Temperatursprung aufrecht erhalten wird.

2. Vorrichtung zur Durchführung des Verfahrens nach Anspruch 1, g e k e n n z e i c h n e t   d u r c h   folgende Merkmale:

   a) Es ist ein geschlossener Gaskreislauf, insbesondere unter leichtem Überdruck vorhanden, der teilweise in einem thermostatisierten Kühlbad (1) verläuft, wobei

31.03.83 Nw/Pa

-11- 24.673.0

insbesondere eine Wasserdampfdruck-Ausgleichsvorrichtung (2, 3) und/oder ein Beruhigungsgefäß (4) im Kühlbad angeordnet sind.

b) In das Beruhigungsgefäß (4) ragt von außerhalb des Kühlbades ein beheizbares Aufwärmrohr (12) hinein, welches im Inneren des Beruhigungsgefäßes (4) endet und als
Auslaß für das Trägergas dient.

3. Vorrichtung nach Anspruch 2, g e k e n n z e i c h -
n e t   d u r c h   folgende Merkmale:

a) Das Aufwärmrohr (12) ist durch einen in seinem Inneren verlaufenden Heizdraht (15) beheizbar.

b) Der Heizdraht (15) verläuft im wesentlichen ohne Wandberührung in der Mitte des Aufwärmrohres (12).

4. Vorrichtung nach Anspruch 3, g e k e n n z e i c h -
n e t   d u r c h   folgendes Merkmal:

a) Der Heizdraht (15) wird durch kleine Distanzstücke,
zumindest im Bereich unterhalb des Kühlmittelspiegels,
auf Abstand von der Wand des Aufwärmrohres (12) gehalten.

5. Vorrichtung nach Anspruch 3 oder 4, g e k e n n -
z e i c h n e t   d u r c h   folgendes Merkmal:

a) Der Heizdraht (15), bzw. seine Zuleitung, wird an
einer Abzweigung (14) oberhalb des Kühlmittelspiegels
dicht durch die Wand des Aufwärmrohres (12) nach aussen geführt.

6. Vorrichtung nach einem der vorhergehenden Ansprüche,
g e k e n n z e i c h n e t   d u r c h   folgendes
Merkmal:

a) Die Durchführung (13) des Aufwärmrohres (12) in das
Beruhigungsgefäß (4) ist möglichst kurz und/oder schlecht
wärmeleitend.

83 P 6716 E  0126282

-12-                    24.673.0

7. Vorrichtung nach einem der vorhergehenden Ansprüche,
g e k e n n z e i c h n e t   d u r c h   folgendes
Merkmal:

a) Das Aufwärmrohr (12) besteht aus einem schlecht wärmeleitenden Material.

8. Vorrichtung nach einem der vorhergehenden Ansprüche,
g e k e n n z e i c h n e t   d u r c h   folgendes
Merkmal:

a) Der Heizdraht (15) ragt etwas über das Ende des Aufwärmrohres (12) hinaus in das Innere des Beruhigungsgefäßes (4).

9. Vorrichtung nach einem der vorhergehenden Ansprüche,
g e k e n n z e i c h n e t   d u r c h   folgendes
Merkmal:

a) Im Kühlbad (1) sind nebeneinander eine Kühlschlange (6)
eine Wasserdampfdruck-Ausgleichsvorrichtung (2, 3) und
ein Beruhigungsgefäß (4) mit einem als Auslaß dienenden
Aufwärmrohr (12) angeordnet, welche nacheinander vom
Trägergas durchströmbar sind.

10. Vorrichtung nach einem der vorhergehenden Ansprüche,
g e k e n n z e i c h n e t   d u r c h   folgende
Merkmale:

a) Die Wasserdampfdruck-Ausgleichsvorrichtung (2, 3) besteht aus einem mit feuchtem Pulver (3), vorzugsweise
$Al_2O_3$, gefüllten Behälter (2), in den oberhalb der Füllung (3) ein Gaseinlaßrohr (7) mündet.

b) Der Gasauslaß der Wasserdampfdruck-Ausgleichsvorrichtung (2, 3) besteht aus einem in der Füllung (3) endenden Rohr (8) welche perforiert (9) ist.

11. Vorrichtung nach einem der vorhergehenden Ansprüche,

-13-                24.673.0

g e k e n n z e i c h n e t   d u r c h   folgendes
Merkmal:

a) Der Gaseinlaß des Beruhigungsgefäßes (4) besteht aus einem perforierten (10) Rohr (8) welches etwas oberhalb des Bodens in das Beruhigungsgefäß (4) hineinragt.

12. Vorrichtung nach einem der vorhergehenden Ansprüche, g e k e n n z e i c h n e t   d u r c h   folgendes Merkmal:

a) Außerhalb des Kühlbades (1) befinden sich Hilfsgeräte zum Betrieb des Kreislaufes, sowie eine oder mehrere Anschlußstellen (17) für Feuchtemeßgeräte.

13. Vorrichtung nach Anspruch 12, g e k e n n z e i c h - n e t   d u r c h   folgendes Merkmal:

a) Als Hilfsgeräte zum Betrieb des Kreislaufes dienen eine Pumpe (5) mit nachgeordnetem Dämpfungsgefäß (20) zur Pulsationsdämpfung, ein Ausgleichsgefäß (19) zur Aufrechterhaltung eines leichten Überdruckes und Meßgeräte für Durchfluß (18) und Druck.

0126282